# EUROPÄISCHE PATENTSCHRIFT

(11) **EP 4 311 561 B1**
(45) Veröffentlichungstag und Bekanntmachung des Hinweises auf die Patenterteilung: **18.03.2026**
(21) Anmeldenummer: 23187141.9
(22) Anmeldetag: 24.07.2023
(51) Int. Cl.: A61M 1/00, A61M 25/00

(54) **SONDENSYSTEM FÜR DIE ENDOLUMINALE UNTERDRUCKTHERAPIE**
PROBE SYSTEM FOR ENDOLUMINAL NEGATIVE PRESSURE THERAPY
SYSTÈME DE SONDE POUR THÉRAPIE ENDOLUMINALE PAR PRESSION NÉGATIVE

(30) Priorität: 25.07.2022 DE 102022118531
(43) Veröffentlichungstag der Anmeldung: 31.01.2024
(73) Patentinhaber: ATMOS MedizinTechnik GmbH & Co. KG, 79853 Lenzkirch (DE)
(72) Erfinder: Eckert, Patrick, 79809 Weilheim-Remetschwiel (DE); Weinmann, Theron, 79777 Ühlingen-Birkendorf (DE)
(74) Vertreter: Westphal, Mussgnug & Partner Patentanwälte mbB

(56) Entgegenhaltungen:
- DE-A1- 102016 114 819
- US-A1- 2013 211 385
- US-A1- 2014 052 111
- US-A1- 2019 307 933
- US-A1- 2020 330 652
- US-A1- 2021 260 260

## Beschreibung

Unterdrucktherapie, die auch unter der Bezeichnung Vakuumtherapie bekannt ist, ist eine Behandlungsform, bei der im Bereich einer chronischen oder akuten Wunde oder einer entzündeten Stelle ein Unterdruck angelegt und meistens über längere Zeit hinweg kontinuierlich oder periodisch aufrecht erhalten oder aufgebaut wird, was insbesondere zu einer verbesserten Wundheilung beitragen kann, beispielsweise dadurch, dass abgegebene Wundsekrete effektiv abgesaugt werden. Einen Überblick über Anwendungsmöglichkeiten der Unterdrucktherapie und Unterdrucktherapiegeräte findet man beispielsweise in der DE 10 2016 114 819 A1.

Die Unterdrucktherapie wird in zunehmendem Maße auch endoluminal, also im Inneren einer Körperhöhle eingesetzt, beispielsweise im Lumen des Gastrointestinaltraktes. Auch derartige Anwendungen sind in der bereits genannten DE 10 2016 114 819 A1 ausführlich beschrieben. Bei der endoluminalen Unterdrucktherapie wird ein Sondenkörper, der mit einem Schlauch verbunden ist, in das Lumen eingeführt und an die zu behandelnde Stelle gebracht. Um eine umfassende Abfuhr von Wundsekreten zu ermöglichen und gleichzeitig eine schonende Behandlung des die Lumenwand bildenden Gewebes zu gewährleisten, ist dabei nach dem Stand der Technik am Sondenkörper ein Fluidsammelkörper angeordnet, der durch einen Schwammköper gebildet wird.

In der medizinischen Praxis zeigt sich allerdings, dass mit derartigen Schwammkörpern ein erhebliches Risiko einher geht, dass es zu Verwachsungen des Schwammkörpers mit dem die Lumenwand bildenden Gewebe kommt. Dies begrenzt die Zeit, die ein gegebener Sondenkörper im Lumen verbleiben kann und schafft die Notwendigkeit zu einem mehrfachen Wechsel des Sondensystems für die endoluminale Unterdrucktherapie im Verlauf der Wundheilung einer endoluminalen Wunde, die sich über mehrere Wochen hinziehen kann.

Ein Sondensystem aus dem Stand der Technik offenbart die US 2013/0211385 A1. Weitere bekannte Sondensysteme werden beispielsweise in der US 2019/ 0307933 A1, der US 2020/0330652 A1, der US 2014/0052111 A1 und der US 2021/0260260 A1 beschrieben.

Die Aufgabe der Erfindung besteht daher darin, ein verbessertes Sondensystem für die endoluminale Unterdrucktherapie bereitzustellen, das langfristig im Lumen verbleiben kann. Diese Aufgabe wird gelöst durch ein Sondensystem für die endoluminale Unterdrucktherapie mit den Merkmalen des Patentanspruchs 1. Vorteilhafte Weiterbildungen der Erfindung sind Gegenstand der abhängigen Patentansprüche.

Das erfindungsgemäße Sondensystem für die endoluminale Unterdrucktherapie weist einen Sondenschlauch und einen mit dem Sondenschlauch verbundenen Sondenkörper auf. Der Sondenkörper hat einen Grundkörper mit einem inneren Lumen und mit mindestens einer Öffnung, die von dem inneren Lumen ausgeht und durch die Außenwand des Grundkörpers zu einer Außenseite des Grundkörpers führt.

In vielen Fällen ist es dabei vorteilhaft, wenn der Grundkörper zylinderförmig ist. Das Lumen kann beispielsweise durch eine koaxial zu der Zylinderachse des zylindrischen Grundkörpers verlaufende Öffnung oder Bohrung gebildet werden; die Öffnung(en), die das innere Lumen und die Außenseite des Grundkörpers führen, können dann vorzugsweise die Außenwand, die durch den Zylindermantel gebildet wird, senkrecht zur Zylinderachse durchsetzen. Bevorzugt ist es dabei, wenn mehrere Öffnungen vorhanden sind, die in unterschiedliche Richtungen weisen, so dass beim Erzeugen von Unterdruck eine Saugrichtung in mehrere Richtungen entsteht.

Erfindungswesentlich ist, dass der Sondenkörper an der Außenseite des Grundkörpers, insbesondere an seiner äußeren Mantelfläche mehrere stabförmige und/oder mindestens eine lamellenförmige Strukturen aufweist. In manchen Ausführungsformen der Erfindung können dabei diese Strukturen ebenfalls einen Kanal mit einer Austrittsöffnung aufweisen, wobei der Kanal mit dem inneren Lumen des Grundkörpers kommuniziert.

Dem Begriff "stabförmig" unterfallen dabei insbesondere Strukturen, deren Länge, gemessen von ihrem Ausgangspunkt am Grundkörper hin zu ihrem dem Grundkörper gegenüber liegenden Ende ein Mehrfaches, bevorzugt mehr als das Fünffache der größten Ausdehnung ihres Querschnitts senkrecht zu dieser Längsrichtung ist. Der Querschnitt kann sich dabei entlang der Längsrichtung der Struktur ändern. Besonders bevorzugt sind Ausführungsformen mit einem kreisförmigen Querschnitt, so dass stabförmige Strukturen insbesondere die Geometrie eines Zylinders, eines Kegelstumpfes oder eines Kegels aufweisen können, dessen Höhe jeweils ein Mehrfaches, bevorzugt mehr als das Fünffache seines größten Durchmessers beträgt.

Dem Begriff "lamellenförmig" unterfallen dabei insbesondere Scheiben und bandförmige Strukturen, wenn deren Höhe, gemessen ausgehend von ihrem Ausgangspunkt am Grundkörper hin zu ihrem dem Grundkörper gegenüber liegenden Ende, ein Mehrfaches, insbesondere mehr als das Fünffache ihrer Dicke (die insbesondere durch den Abstand zwischen ihren größten Oberflächen definiert ist) beträgt.

Diese stabförmigen und/oder lamellenförmigen Strukturen stellen sicher, dass die jeweilige Wand der Körperöffnung oder Körperhöhle, in die sie eingeführt sind, vom Grundkörper beabstandet ist und nicht durch Unterdruck in Öffnungen des Grundkörpers des Sondenkörpers eingesaugt werden kann.

Gleichzeitig handelt es sich aber auch dann, wenn mehrere solche Strukturen vorhanden sind, um Einzelstrukturen, die voneinander beabstandet sind, so dass anders als bei den schwammartigen Strukturen aus dem Stand der Technik das Risiko von Verwachsungen zwischen Sondenkörper und Gewebe drastisch reduziert ist.

Die Effizienz des Entfernens von Wundsekreten ist dabei überraschend hoch, obwohl keine Aufnahme der Wundsekrete durch einen Schwamm erfolgt, was vermutlich darauf zurückzuführen ist, dass der Schwamm einen relativ hohen Saugwiderstand darstellt, so dass die Absaugung effizienter wird.

Besondere Vorteile bringt der Sondenkörper des erfindungsgemäßen Sondensystems dann mit sich, wenn dieses nicht nur zur Unterdrucktherapie eingesetzt wird sondern auch zur Einbringung von Spülflüssigkeiten oder Medikamenten zum Säubern der Wunde oder zum Lösen von Verstopfungen. Durch die Kombination von Spülen und Saugen wird eine suffiziente Drainage von Wundsekreten gewährleistet. Bei den Schwammsystemen ist diese Kombination nicht vorhanden wodurch diese schnell verstopfen.

Mit dem Sondenkörper des erfindungsgemäßen Sondensystems kann man die Flüssigkeit unmittelbar aus den Öffnungen des Grundkörpers austreten lassen.

Zudem deuten die bisherigen Erfahrungen darauf hin, dass durch die Strukturen am erfindungsgemäßen Sondenkörper eine den Heilungsverlauf fördernde Stimulation des Gewebes bewirkt wird.

Besonders bevorzugt ist es, wenn der Grundkörper und der Sondenschlauch einstückig ausgeführt oder stoffschlüssig miteinander verbunden sind. Auf diese Weise kann die Integrität des Sondensystems auch beim Einführen in das oder Entfernen aus dem Lumen besonders sicher gewährleistet werden. Aus demselben Grund ist auch eine einstückige Ausführung oder stoffschlüssige Verbindung zwischen Grundkörper und den Strukturen bevorzugt.

Die Strukturen verlaufen erfindungsgemäß die senkrecht vom Grundkörper weg und/oder stehen senkrecht zur Außenseite des Grundkörpers, also zu dessen Außenwand, durch die die Öffnungen führen, wenn der Sondenkörper nicht in eine Körperhöhle eingeführt ist.

Da die Strukturen erfindungsgemäß ringförmig oder spiralförmig auf der Außenwand des Grundkörpers angeordnet sind, stellen sie eine gleichzeitige Beabstandung des Grundkörpers von der Wand der Körperöffnung, in die der Sondenkörper eingeführt ist, in allen Richtungen, die senkrecht zur Verlaufs- oder Erstreckungsrichtung des Grundkörpers stehen, sicher. Dies reduziert die Wahrscheinlichkeit für das Verstopfen von Öffnungen und erhöht die Wahrscheinlichkeit, dass unverstopfte Öffnungen vorhanden sind.

Besonders bevorzugt ist es, wenn die Strukturen flexibel und/oder elastisch sind. Dafür gibt es insbesondere zwei Gründe: Erstens können sie sich in diesem Fall beim Anlegen von Unterdruck verformen und gegebenenfalls sogar am Grundkörper anliegen, was die Absaugeffizienz verbessert, aber weiterhin sicherstellt, dass die Körperhöhle nicht vollständig bis auf die Oberfläche des Grundkörpers kollabiert, weil die Körperhöhle noch immer durch die dann ihrerseits am Grundkörper liegenden Strukturen vom Grundkörper auf Abstand von Diesem gehalten wird. Zweitens scheinen die bisherigen Erfahrungen darauf hinzudeuten, wird auf diese Weise schonend eine besonders effiziente Stimulation des Gewebes erreicht, die die Wundheilung fördert.

Dadurch, dass die Länge, also der Abstand zwischen ihrem Ausgangspunkt am Grundkörper und dem dem Grundkörper gegenüber liegenden Ende, der Strukturen variiert, kann der Sondenkörper hinsichtlich seiner Geometrie angepasst und so beispielsweise besonders leicht einführbar in die Körperhöhle gestaltet werden.

Gemäß der Erfindung ist vorgesehen, dass der Sondenschlauch ein zweilumiger Schlauch ist. In dieser Ausgestaltung kann in besonders einfacher Weise zusätzlich zur Unterdrucktherapie-Funktion eine Spülfunktion realisiert werden, bei der eine Spülflüssigkeit, z.B. eine NaCl-Lösung, über eines der Lumen zugeführt wird und über das andere Lumen abgesaugt wird. Ein medizinisch wichtiger Vorteil vom zweilumigen System ist, dass bereits abgesaugtes Sekret was im Sauglumen liegt durch den Spülvorgang nicht wieder zurück in die Körperöffnung, z.B. einen Darm, gelangt. Zudem ist dadurch die Spüldauer beim 2-lumigen System kürzer.

Bei der grundsätzlich ebenfalls möglichen, aber nicht zur beanspruchten Erfindung gehörenden Verwendung eines Sondenschlauchs mit nur einem Lumen muss beim Umschalten zwischen dem Spülbetrieb und dem Unterdruckbetrieb jeweils gewährleistet werden, dass die jeweils nicht verwendete Flüssigkeits- bzw. Vakuumquelle vollständig gegen den Sondenschlauch abgedichtet ist, um die ordnungsgemäße Ausführung der aktuell durchzuführenden Aktion zu gewährleisten, während eine solche Abdichtung bei einem zweilumigen Sondenschlauch nicht bei jedem Umschalten neu erfolgen muss, sondern nur einmal beim Anschluss des Sondenschlauchs an die jeweiligen Quellen.

Erfindungsgemäß haben die beiden Lumen des zweilumigen Schlauchs unterschiedlich große Querschnittsflächen und die Spülflüssigkeit wird über das Lumen mit der kleineren Querschnittsfläche zugeführt. Besonders bevorzugt wird eine Ausführungsform, bei der die Querschnittsfläche des Lumens, das zum Absaugen verwendet wird, möglichst groß ist. Der Querschnitt, auf den dabei Bezug genommen wird, ist ein Schnitt senkrecht zur Verlaufsrichtung des Sondenschlauchs.

Besonders einfach herzustellen ist ein solcher zweilumiger Sondenschlauch, wenn erfindungsgemäß der Querschnitt beider Lumen des zweilumigen Schlauchs kreisförmig ist.

Wenn sowohl die Spülfunktion als auch die Unterdrucktherapiefunktion realisiert werden sollen kann es vorteilhaft sein, auch im Grundkörper der Sonde zwei Lumen vorzusehen, was insbesondere auch eine gleichzeitige Anwendung beider Funktionen ermöglicht. Wenn sowohl das Spülen als auch das Absaugen über den gesamten Umfang des Grundkörpers hinweg erfolgen sollen, können diese beispielsweise als zwei einander umlaufende Wendeln oder Doppelhelix gestaltet sein; sie können aber auch einfach parallel zueinander verlaufen. Dementsprechend haben dann die Öffnungen im Grundkörper in diesem Fall entweder nur eine Spülfunktion oder nur eine Absaugfunktion, während sie sonst ihre Funktion in Abhängigkeit vom jeweils aktiven Betriebsmodus ändern.

Für eine exakte Positionierung des Sondenkopfes ist es vorteilhaft, wenn auf der Außenseite des Sondenschlauchs eine Kennzeichnung angebracht ist, so dass die Position des Sondensystems in einem Lumen, in das es eingeführt ist, abgelesen werden kann.

Eine alternativ oder zusätzlich vorzunehmende Maßnahme, die die exakte Positionierung des Sondenkörpers im Inneren des Lumens ermöglicht, besteht darin, dass am Sondenschlauch ein Röntgenkontraststreifen angebracht ist und/oder dass das Material, aus dem der Sondenschlauch und/oder der Sondenkörper bestehen, mit einem Röntgenkontrastmittel versetzt ist. Dadurch wird es möglich, durch eine Röntgenaufnahme die Position des Sondenkörpers im Lumen genau zu bestimmen.

Dadurch, dass das innere Lumen des Grundkörpers auf der der Verbindungsstelle zum Sondenschlauch gegenüber liegenden Seite mit einem Stopfen verschlossen ist, wird vermieden, dass am distalen Ende des Grundkörpers eine Öffnung entsteht, in die Wandabschnitte der Körperhöhle, in die der Sondenkörper eingebracht ist, oder größere Partikel, die darin vorhanden sind, in das innere Lumen eingesaugt werden und/oder dieses verstopfen. Grundsätzlich könnte man dies auch durch ein in distaler Richtung durch eine Wand verschlossenes Lumen erreichen, was aber dazu führt, dass bei einer Längenanpassung des Grundkörpers die oben erwähnten Probleme auftreten. Somit trägt der Stopfen auch dazu bei, eine Längenanpassung des Sondenkörpers zu ermöglichen.

Wenn an der der Verbindungsstelle zum Sondenschlauch gegenüber liegenden, also der distalen Seite des Grundkörpers eine Schlaufe angeordnet ist, kann die Sonde mit Hilfe endoskopischer Werkzeuge leicht eingeführt werden.

Besonders vorteilhaft ist es, wenn der Sondenkörper und der Sondenschlauch aus Silikon bestehen. Dieses Material weist eine sehr gute Biokompatibilität und eine hohe chemische Beständigkeit gegen verschiedene Medien auf. Gleichzeitig ist es weich und flexibel und reißfest.

Die Erfindung wird nachfolgend anhand von Figuren, die Ausführungsbeispiele zeigen näher erläutert. Es zeigt:
- Fig. 1a:: Eine Aufsicht auf ein erstes Ausführungsbeispiel eines Sondensystems;
- Fig. 1b:: einen Querschnitt durch das Sondensystem aus Figur 1a;
- Fig. 2a:: eine Aufsicht auf einen Sondenkörper zweites Ausführungsbeispiel eines Sondensystems;
- Fig. 2b:: eine vergrößerte Darstellung des Stopfens des Sondenkörpers aus Fig. 2a;
- Fig. 3a:: einen Sondenkörper eines dritten Ausführungsbeispiels eines Sondensystems;
- Fig. 3b:: einen Sondenkörper eines vierten Ausführungsbeispiels eines Sondensystems;
- Fig. 3c:: einen Sondenkörper eines fünften Ausführungsbeispiels eines Sondensystems;
- Fig. 3d:: einen Sondenkörper eines sechsten Ausführungsbeispiels eines Sondensystems;
- Fig. 3e:: einen Sondenkörper eines siebten Ausführungsbeispiels eines Sondensystems;
- Fig. 3f:: einen Sondenkörper eines achten Ausführungsbeispiels eines Sondensystems;
- Fig. 3g:: einen Sondenkörper eines neunten Ausführungsbeispiels eines Sondensystems;
- Fig. 4a:: einen Querschnitt durch eine erste Variante eines zweilumigen Sondenschlauchs;
- Fig. 4b:: einen Querschnitt durch eine nicht erfindungsgemäße Variante eines zweilumigen Sondenschlauchs; und
- Fig. 4c:: einen Querschnitt durch eine weitere nicht erfindungsgemäße eines zweilumigen Sondenschlauchs.

Für unterschiedliche Darstellungen gleicher Ausführungsformen werden dieselben Bezugszeichen verwendet. Allerdings sind zur Wahrung der Übersichtlichkeit der Figuren nicht immer alle Bezugszeichen angetragen. Insbesondere werden Strukturen oder Komponenten, die in großer Zahl in einer Ausführungsform vorhanden sind -beispielsweise die Öffnungen im Grundkörper und stab- oder lamellenförmige Strukturen- lediglich exemplarisch mit einem für all diese Strukturen oder Komponenten einer gegebenen Ausführungsform gleichen, ihnen zugeordneten Bezugszeichen versehen.

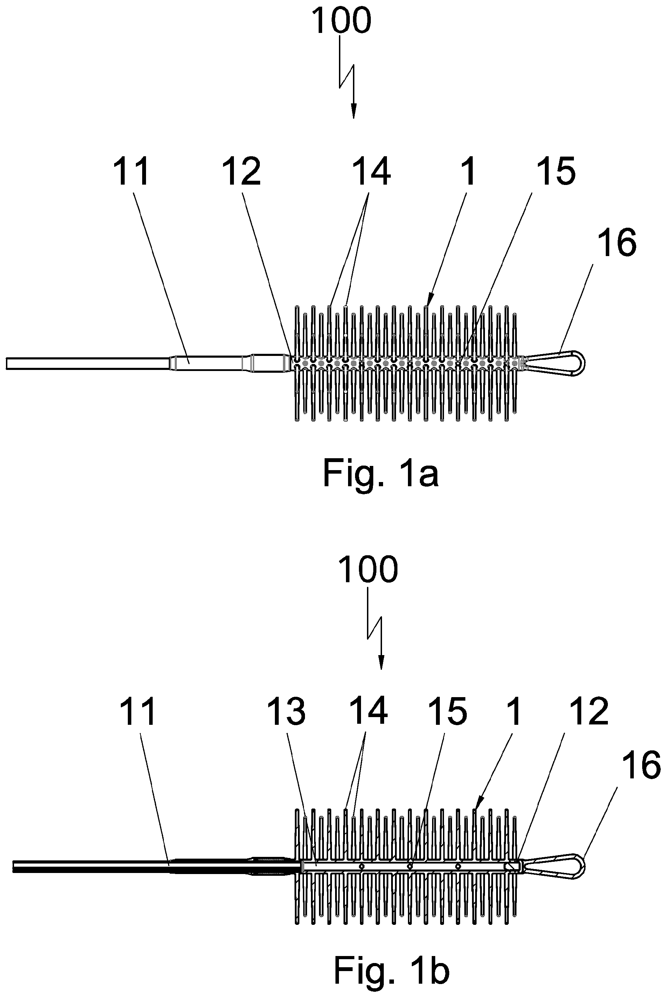

Die Figuren 1a und 1b zeigen zwei Ansichten eines ersten Sondensystems 100. Das Sondensystem 100 weist einen Sondenschlauch 11 auf, der mit dem hier zylinderförmigen Grundkörper 12 eines Sondenkörpers 1 verbunden ist. Das Lumen des Sondenschlauchs 11 kommuniziert, wie die Figur 1b verdeutlich, mit einem koaxial zur Zylinderachse des zylinderförmigen Grundkörpers 12, welche dessen Erstreckungsrichtung definiert, verlaufenden inneren Lumen 13 des Grundkörpers 12, der am distalen, dem Sondenschlauch 11 gegenüber liegenden Ende verschlossen ist. An der Außenseite des Grundkörpers 12 sind eine Vielzahl von stabförmigen Strukturen 14, die senkrecht, also in radialer Richtung zur Zylinderachse des zylinderförmigen Grundkörpers 12, von der Außenseite des Grundkörpers 12 ausgehen, angeformt. Die stabförmigen Strukturen 14 sind in mehrere Gruppen gruppiert, wobei die Ausgangspunkte der stabförmigen Strukturen 14 einer gegebenen Gruppe an der Oberfläche des Grundkörpers 12, jeweils auf einem Ring liegen, so dass sie ringförmig angeordnet sind. Ausgehend vom inneren Lumen 13 des Grundkörpers 11 führen Öffnungen 15 auf seine Außenseite. Dementsprechend kann über das Lumen des Sondenschlauchs 11, das innere Lumen 13 und die Öffnungen 15 Fluid aus der Umgebung des Sondenkörpers 1 abgesaugt oder in die Umgebung des Sondenkörpers 1 eingebracht werden. Am distalen Ende des Grundkörpers 12 ist darüber hinaus eine Schlaufe 16 angeordnet, um die Platzierung des Sondenkörpers 1 zu erleichtern.

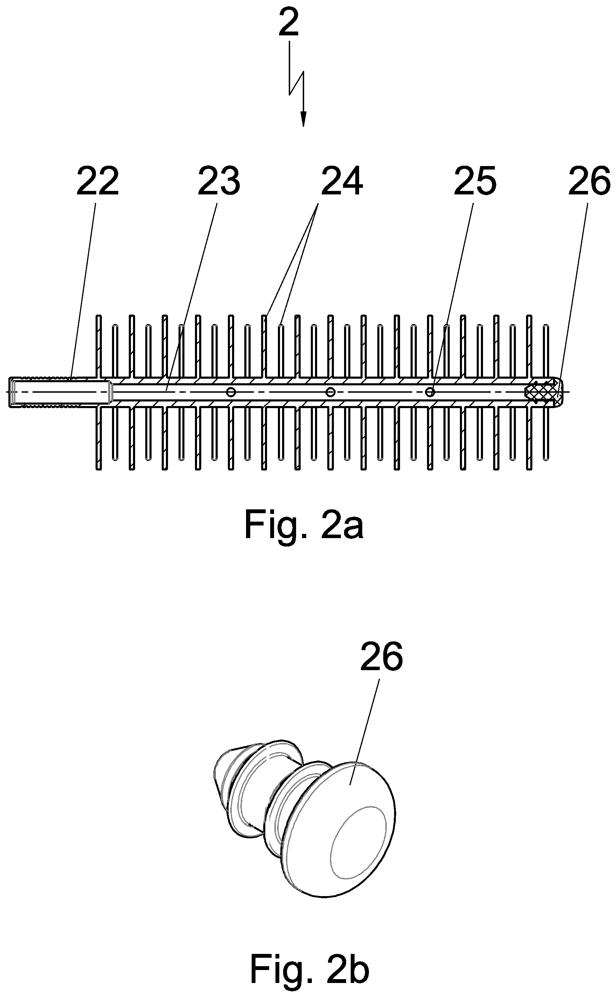

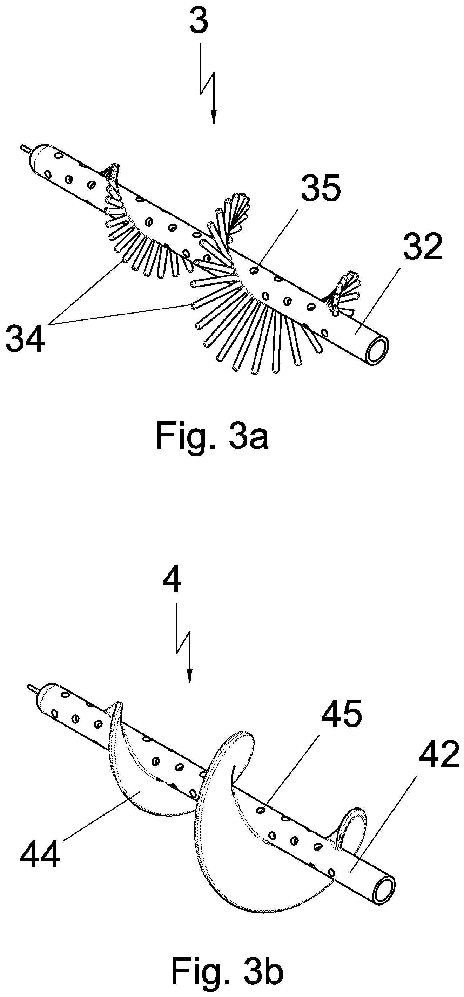

Die in den Figuren 2a und b sowie 3a bis 3g gezeigten Sondenkörper 2,3,4,5,6,7,8,9 können zur Bildung eines Sondensystems 100 jeweils mit einem Sondenschlauch auf die in Figur 1a und 1b dargestellte Weise verbunden werden. Deshalb ist der Sondenschlauch, der zu den jeweiligen Sondensystemen gehört, in diesen Figuren nicht dargestellt.

Der in der Figur 2a gezeigte Sondenkörper 2 mit zylinderförmigem Grundkörper 22 mit innerem Lumen 23 und stabförmigen Strukturen 24 und Öffnungen 25 , unterscheidet sich von dem Sondenkörper 1 durch seine Länge und die Anzahl der an ihm angeordneten Ringe von stabförmigen Strukturen 24. Tatsächlich kann der Sondenkörper 2 durch Kürzen des Sondenkörpers 1 erhalten werden; die dadurch am distalen Ende des Sondenkörpers 2 entstehende Öffnung ist mit dem in Figur 2b vergrößert dargestellten Stopfen 26 verschlossen.

Die in den Figuren 3a bis 3g gezeigten Sondenkörper 3,4,5,6,7, 8,9 mit zylinderförmigen Grundkörpern 32,42,52,62,72,82,92 mit in den Figuren nicht dargestelltem innerem Lumen und Öffnungen 35,45,55,65,75,85,95 unterscheiden sich hinsichtlich der Form und Verteilung der auf ihnen angeordneten stabförmigen und/oder lamellenförmigen Strukturen 34,44,54,64,74,84. Die jeweils bevorzugte Form und Verteilung der Strukturen 34,44,54,64,74,84 kann dabei insbesondere von anatomischen Gegebenheiten des Körperhohlraums und/oder der zu behandelnden Wunde abhängen.

In Figur 3a sind stabförmige Strukturen 34, deren Länge von proximal nach distal zunächst kontinuierlich zunimmt und dann wieder kontinuierlich abnimmt, spiralförmig um den Grundkörper 32 herum angeordnet.

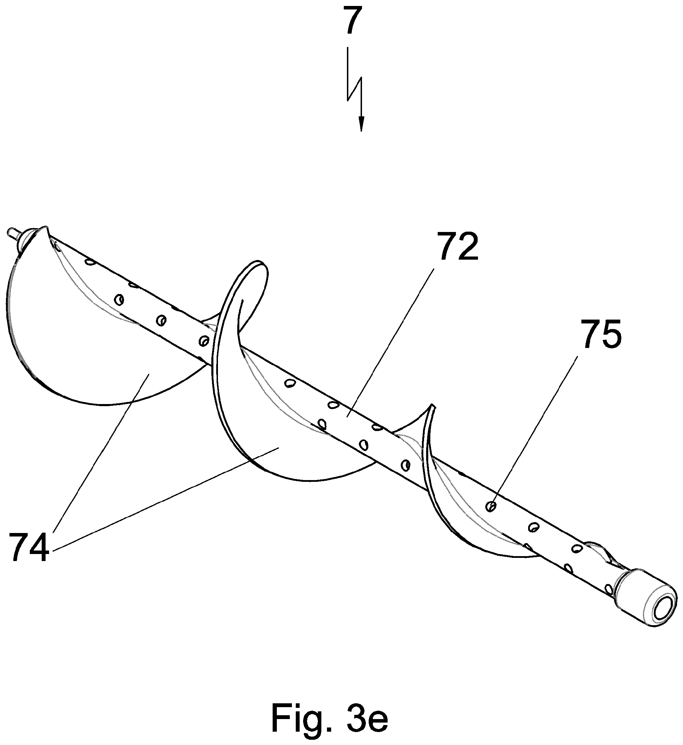

In den Figuren 3b und 3e ist eine lamellenförmige Struktur 44 bzw. 74 jeweils spiralförmig um den Grundkörper 42 bzw. 72 herum gewendelt angeordnet, wobei sich die Zahl der Wendeln unterscheidet und die Höhe der Wendeln beim Sondenkörper 4 von proximal nach distal zunimmt, während sie beim Sondenkörper 7 von proximal nach distal abnimmt.

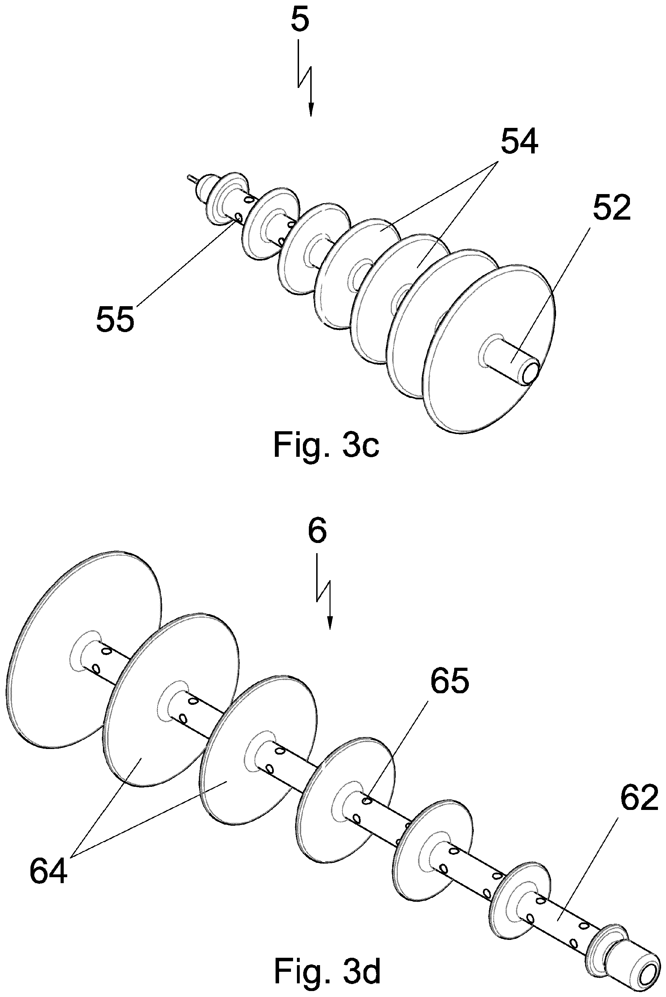

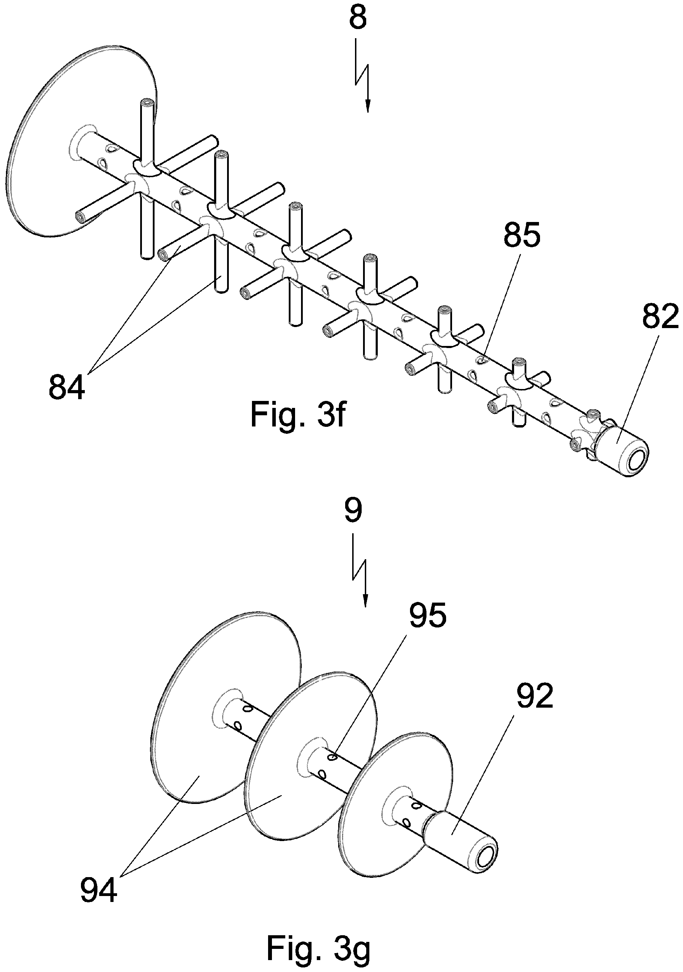

In den Figuren 3c,3d und 3g sind jeweils mehrere lamellenförmige Strukturen 54,64,94 in Gestalt von Scheiben unterschiedlichen Durchmessers konzentrisch mit der Zylinderachse des zylindrischen Grundkörpers 52,62,92 an diesem angeformt. Dabei nimmt in Figur 3c der Durchmesser der lamellenförmigen Strukturen 54 von proximal nach distal stark zu, in Figur 3d der Durchmesser der lamellenförmigen Strukturen 64 von proximal nach distal stark ab und in Figur 3g der Durchmesser der lamellenförmigen Strukturen 94 von proximal nach distal leicht ab. Zudem variieren der Abstand und die Anzahl der lamellenförmigen Strukturen 54,64 bzw. 94.

Der in Figur 3f dargestellte Sondenkörper 8 hat an seinem proximalen Ende eine lamellenförmige, als Scheibe ausgeführte Struktur 84 sowie eine Vielzahl von stabförmigen Strukturen 84, von denen jeweils vier eine an einer gegebenen Stelle des Grundkörpers 82 kreuzförmig angeordnete Gruppe bilden. Dabei nimmt die Länge der stabförmigen Strukturen 84 der jeweiligen Gruppe von proximal nach distal kontinuierlich ab.

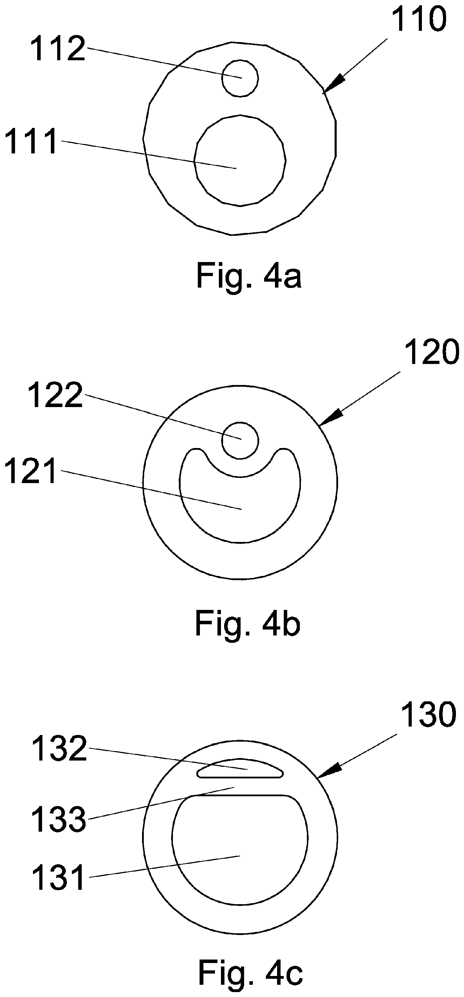

Die Figuren 4a bis 4c zeigen drei Querschnitte von zweilumigen Sondenschläuchen 110,120,130, die verwendet werden können, um einen Spül- und Unterdruckbetrieb des Sondensystems einfach zu realisieren, indem für jede Betriebsart ein eigenes Lumen bereitgestellt wird, wobei nur die in Figur 4a gezeigte Ausführungsform zur beanspruchten Erfindung gehört. Die Sondenschläuche 110,120,130 sind dementsprechend jeweils zweilumig mit Lumen 111,112; 121,122 bzw. 131,132 ausgeführt, wobei die für den Spülbetrieb vorgesehenen Lumen 112,122,132 jeweils einen kleineren Querschnitt aufweisen. Während die Ausführungsform gemäß Figur 4a besonders einfach herzustellen ist, ist in der Ausführungsform der Figur 4c der Querschnitt des Lumens 131 maximiert, was Vorteile für die Absaugung mit sich bringt.

Konkret ist in Figur 4a der Querschnitt beider Lumen 111,112 des zweilumigen Schlauchs kreisförmig, während in der nicth zur Erfindung gehörenden Figur 4b der Querschnitt eines der Lumen 122 des zweilumigen Schlauchs kreisförmig und der Querschnitt des anderen Lumens 121 des zweilumigen Schlauchs halbmondförmig ist, wobei die konkave Seite des Halbmonds dem kreisförmigen Lumen 122 zugewandt ist. In der nicht zur beanspruchten Erfindung gehörenden Ausführungsform der Figur 4c sind die beiden Lumen 131,132 des zweilumigen Schlauchs im Querschnitt betrachtet durch eine gerade Wand 133 voneinander getrennt, wodurch ein besonders großes Volumen für das Absaugen erzeugt werden kann.

### Bezugszeichenliste

- 1,2,3,4,5,6,7,8,9: Sondenkörper
- 11,110,120,130: Sondenschlauch
- 12,22,32,42,52,62,72,82,92: Grundkörper
- 13,23: inneres Lumen
- 14,24,34,44,54,64,74,84,94: Struktur
- 15,25,35,45,55,65,75,85,95: Öffnung
- 16: Schlaufe
- 26: Stopfen
- 100: Sondensystem
- 111,112,121,122,131,132: Lumen
- 133: Wand

## Patentansprüche

1. Sondensystem (100) für die endoluminale Unterdrucktherapie mit einem Sondenschlauch (11,110,120,130) und einem mit dem Sondenschlauch (11,110,120,130) verbundenen Sondenkörper (1,2,3,4,5,6,7,8,9), der einen Grundkörper (12,22,32, 42,52,62,72,82,92) mit einem inneren Lumen (13,23) und mit mindestens einer Öffnung (15,25,35,45,55,65,75,85,95), die von dem inneren Lumen (13,23) durch eine Außenwand des Grundkörpers (12,22,32,42,52,62,72,82,92) zu einer Außenseite des Grundkörpers (12,22,32,42,52,62,72,82,92) führt, aufweist, wobei der Sondenkörper (1,2,3,4,5,6,7,8,9) an der Außenseite des Grundkörpers (12,22,32,42,52,62,72, 82,92) mehrere stabförmige Strukturen (14,24,34,84) und/oder mindestens eine lamellenförmige Struktur (44,54, 64,74,94) aufweist,
wobei die Strukturen (14,24,34,44,54,64,74,84,94) senkrecht zur Außenwand des Grundkörpers (12,22,32,42,52,62, 72,82) stehen,
wobei
die Strukturen (14,24,34,44,54,64,74,84,94) ringförmig oder spiralförmig auf der Außenwand des Grundkörpers (12,22,32,42,52,62,72,82,92) angeordnet sind,
und wobei der Sondenschlauch (11,110,120,130) ein zweilumiger Schlauch ist, wobei der Querschnitt der beiden Lumen (111,112,121,122,131,132) des zweilumigen Schlauchs eine unterschiedliche Querschnittsfläche aufweist und die Spülflüssigkeit über das Lumen mit der kleineren Querschnittsfläche zugeführt wird, und wobei der Querschnitt beider Lumen (111,112) des zweilumigen Schlauchs kreisförmig ist,
und wobei das innere Lumen (13,23) des Grundkörpers (12,22, 32,42,52,62,72, 82,92) auf der der Verbindungsstelle zum Sondenschlauch (11,110,120,130) gegenüber liegenden Seite mit einem Stopfen (26) verschlossen ist.

2. Sondensystem (100) nach Anspruch 1,
**dadurch gekennzeichnet, dass** der Grundkörper (12,22,32,42,52,62,72,82,92) und der Sondenschlauch (11,110,120,130) einstückig ausgeführt oder stoffschlüssig miteinander verbunden sind.

3. Sondensystem (100) nach einem der Ansprüche 1 oder 2,
**dadurch gekennzeichnet, dass** die Strukturen (14,24,34,44,54,64,74,84,94) flexibel und/oder elastisch sind.

4. Sondensystem (100) nach eine der Ansprüche 1 bis 3,
**dadurch gekennzeichnet, dass** die Länge der Strukturen (14,24,34,44,54,64,74,84,94) variiert.

5. Sondensystem (100) nach einem der Ansprüche 1 bis 4,
**dadurch gekennzeichnet, dass** auf der Außenseite des Sondenschlauchs (11,110,120,130) eine Kennzeichnung angebracht ist, so dass die Position des Sondensystems (100) in einem Lumen einer Körperhöhle, in das es eingeführt ist, abgelesen werden kann.

6. Sondensystem (100) nach einem der Ansprüche 1 bis 5,
**dadurch gekennzeichnet, dass** am Sondenschlauch (11,110,120,130) ein Röntgenkontraststreifen eingebracht ist und/oder dass das Material, aus dem der Sondenschlauch (11,110,120,130) und/oder der Sondenkörper (1,2,3,4,5,6,7,8,9) bestehen, mit einem Röntgenkontrastmittel versetzt ist.

7. Sondensystem (100) nach einem der Ansprüche 1 bis 6,
**dadurch gekennzeichnet, dass** an der der Verbindungsstelle zum Sondenschlauch (11,110,120,130) gegenüber liegenden Seite des Grundkörpers (12,22,32,42, 52,62,72,82,92) eine Schlaufe (15) angeordnet ist.

8. Sondensystem (100) nach einem der Ansprüche 1 bis 7,
**dadurch gekennzeichnet, dass** der Sondenkörper (1,2,3,4,5,6,7,8,9) und der Sondenschlauch (11,110,120,130) aus Silikon bestehen.

## Claims

1. A probe system (100) for endoluminal negative-pressure therapy, comprising a probe tube (11, 110, 120, 130) and a probe body (1, 2, 3, 4, 5, 6, 7, 8, 9) connected to the probe tube (11, 110, 120, 130), wherein the probe body comprises a base body (12, 22, 32, 42, 52, 62, 72, 82, 92) having an inner lumen (13, 23) and at least one opening (15, 25, 35, 45, 55, 65, 75, 85, 95), which leads from the inner lumen (13, 23) through an outer wall of the base body (12, 22, 32, 42, 52, 62, 72, 82, 92) to an outer side of the base body (12, 22, 32, 42, 52, 62, 72, 82, 92), wherein the probe body (1, 2, 3, 4, 5, 6, 7, 8, 9) on the outer side of the base body (12, 22, 32, 42, 52, 62, 72, 82, 92) comprises a plurality of rod-shaped structures (14, 24, 34, 84) and/or at least one lamella-shaped structure (44, 54, 64, 74, 94),
wherein the structures (14, 24, 34, 44, 54, 64, 74, 84, 94) are arranged perpendicularly to the outer wall of the base body (12, 22, 32, 42, 52, 62, 72, 82),
wherein the structures (14, 24, 34, 44, 54, 64, 74, 84, 94) are arranged in a ring-shaped or spiral-shaped manner on the outer wall of the base body (12, 22, 32, 42, 52, 62, 72, 82, 92), and
wherein the probe tube (11, 110, 120, 130) is a dual-lumen tube, wherein the cross sections of the two lumens (111, 112, 121, 122, 131, 132) of the dual-lumen tube have different cross-sectional areas, and wherein the irrigation fluid is supplied via the lumen having the smaller cross-sectional area, and wherein the cross section of both lumens (111, 112) of the dual-lumen tube is circular,
and wherein the inner lumen (13, 23) of the base body (12, 22, 32, 42, 52, 62, 72, 82, 92) is closed by a plug (26) on the side opposite the connection point to the probe tube (11, 110, 120, 130).

2. The probe system (100) according to claim 1,
**characterized in that** the base body (12, 22, 32, 42, 52, 62, 72, 82, 92) and the probe tube (11, 110, 120, 130) are formed in one piece or are materially bonded to one another.

3. The probe system (100) according to one of claims 1 or 2, **characterized in that** the structures (14, 24, 34, 44, 54, 64, 74, 84, 94) are flexible and/or elastic.

4. The probe system (100) according to one of claims 1 to 3, **characterized in that** the length of the structures (14, 24, 34, 44, 54, 64, 74, 84, 94) varies.

5. The probe system (100) according to one of claims 1 to 4, **characterized in that** a marking is applied to the outer side of the probe tube (11, 110, 120, 130), such that the position of the probe system (100) within a lumen of a body cavity into which it is inserted can be read.

6. The probe system (100) according to one of claims 1 to 5, **characterized in that** an X-ray contrast strip is incorporated into the probe tube (11, 110, 120, 130) and/or that the material from which the probe tube (11, 110, 120, 130) and/or the probe body (1, 2, 3, 4, 5, 6, 7, 8, 9) is made is mixed with an X-ray contrast agent.

7. The probe system (100) according to one of claims 1 to 6, **characterized in that** a loop (15) is arranged on the side of the base body (12, 22, 32, 42, 52, 62, 72, 82, 92) opposite the connection point to the probe tube (11, 110, 120, 130).

8. The probe system (100) according to one of claims 1 to 7, **characterized in that** the probe body (1, 2, 3, 4, 5, 6, 7, 8, 9) and the probe tube (11, 110, 120, 130) are made of silicone.

## Revendications

1. Système (100) de sonde pour la thérapie endoluminale par dépression comprenant un tube (11, 110, 120, 130) souple de sonde et un corps (1, 2, 3, 4, 5, 6, 7, 8, 9) de sonde, qui est relié au tube (11, 110, 120, 130) de sonde et qui a un corps (12, 22, 32, 42, 52, 62, 72, 82, 92) de base ayant une lumière (13, 23) intérieure et ayant au moins une ouverture (15, 25, 35, 45, 55, 65, 75, 85, 95), qui va, en traversant une paroi extérieure du corps (12, 22, 32, 42, 52, 62, 72, 82, 92) de base, de la lumière (13, 23) à une face extérieure du corps (12, 22, 32, 42, 52, 62, 72, 82, 92) de base, dans lequel le corps (1, 2, 3, 4, 5, 6, 7, 8, 9) de sonde a, sur la face extérieure du corps (12, 22, 32, 42, 52, 62, 72, 82, 92) de base, plusieurs structures (14, 24, 34, 84) en forme de barrette et/ou au moins une structure (44, 54, 64, 74, 94) en forme de lamelles,
dans lequel les structures (14, 24, 34, 44, 54, 64, 74, 84, 94) sont perpendiculaires à la paroi extérieure du corps (12, 22, 32, 42, 52, 62, 72, 82) de base,
dans lequel les structures (14, 24, 34, 44, 54, 64, 74, 84, 94) sont disposées annulairement ou en forme de spirale sur la paroi extérieure du corps (12, 22, 32, 42, 52, 62, 72, 82, 92) de base,
et dans lequel le tube (11, 110, 120, 130) souple de sonde est un tube souple à deux lumières, dans lequel la section transversale des deux lumières (111, 112, 121, 122, 131, 132) du tube souple à deux lumières a une surface de section transversale différente et le liquide de lavage est apporté par la lumière ayant la surface de section transversale la plus petite et dans lequel la section transversale des lumières (111, 112) du tube souple à deux lumières est circulaire,
et dans lequel la lumière (13, 23) intérieure du corps (12, 22, 32, 42, 52, 62, 72, 82, 92) de base est du côté se trouvant opposé au point de liaison avec le tube (11, 110, 120, 130) souple de sonde, fermé par un bouchon (26).

2. Système de sonde (100) suivant la revendication 1,
**caractérisé en ce que** le corps (12, 22, 32, 42, 52, 62, 72, 82, 92) de base et le tube (11, 110, 120, 130) souple de sonde sont réalisés en une seule pièce ou sont reliés entre eux à coopération de matière.

3. Système de sonde (100) suivant l'une des revendications 1 ou 2,
**caractérisé en ce que** les structures (14, 24, 34, 44, 54, 64, 74, 84, 94) sont souples et/ou élastiques.

4. Système de sonde (100) suivant l'une des revendications 1 à 3,
caractérisé à ce que la longueur des structures (14, 24, 34, 44, 54, 64, 74, 84, 94) varie.

5. Système de sonde (100) suivant l'une des revendications 1 à 4,
**caractérisé en ce que**, sur la face extérieure du tube (11, 110, 120, 130) souple de sonde, est appliquée un repère de manière à pouvoir lire la position du système (100) de sonde dans une lumière d'une cavité du corps, dans laquelle il est introduit.

6. Système de sonde (100) suivant l'une des revendications 1 à 5,
**caractérisé en ce que** sur le tube (11, 110, 120, 130) souple de sonde est appliqué une bande de contraste aux rayons X et/ou **en ce que** le matériau dont le tube (11, 110, 120, 130) souple de sonde et/ou le corps (1, 2, 3, 4, 5, 6, 7, 8, 9) de sonde sont constitués, est mélangé à un agent de contraste aux rayons X.

7. Système de sonde (100) suivant l'une des revendications 1 à 6,
**caractérisé en ce qu'**une boucle (15) est disposée sur la face du corps (12, 22, 32, 42, 52, 62, 72, 82, 92) de base, opposée au point de liaison avec le tube (11, 110, 120, 130) souple de sonde.

8. Système de sonde (100) suivant l'une des revendications 1 à 7,
**caractérisé en ce que** le corps (1, 2, 3, 4, 5, 6, 7, 8, 9) de sonde et le tube (11, 110, 120, 130) souple de sonde sont en silicone.
